(19) Europäisches Patentamt
European Patent Office
Office européen des brevets

(11) EP 3 745 410 A1

(12) EUROPEAN PATENT APPLICATION

(43) Date of publication:
02.12.2020 Bulletin 2020/49

(51) Int Cl.:
G16H 20/60 (2018.01)     G16H 50/20 (2018.01)

(21) Application number: 19176699.7

(22) Date of filing: 27.05.2019

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Riprup Company S.A.
GY1 St. Peter Port (GG)

(72) Inventors:
• Bissen, Monique
75175 Pforzheim (DE)
• Schucker, Josef
6622 Ronco Sopra Ascona (CH)

(74) Representative: Wittmann, Günther
Patentanwaltskanzlei Wittmann
Frans-Hals-Straße 31
81479 München (DE)

(54) SELF-LEARNING METHOD OF HYDRATING A HUMAN

(57) The invention discloses a method of monitoring a beverage consumption of a human implemented by a computer, comprising the following steps:
- dividing a timespan of a day into a plurality of time intervals;
- estimating the estimated hydration loss during each of the time intervals based on the activity of the human, the weight of the human, the temperature in the surrounding of the human and the humidity in the surrounding of the human;
- evaluating for each of the time intervals the volume of beverage consumed by the human;
- estimating for each of the time intervals an effective hydration loss of the human based on the estimated hydration loss and the volume of beverage consumed by the human;
- defining an euhydration threshold depending on the weight of the human, wherein the euhydration threshold indicates that the hydration of the human is at the lower limit of euhydration;
- defining an euhydration warning threshold depending on the weight of the human, wherein the euhydration warning threshold indicates an effective hydration loss of the human between average euhydration and the euhydration threshold; and
- sending the human a request to drink a first predetermined amount of beverage, when the effective hydration loss of the human within the at least one time interval exceeds the euhydration warning threshold.

In one embodiment the invention defines a plurality beverage consumer clusters based on hydration development of each of the beverage consumer, based on the physical conditions and/or location of each of the beverage consumer, based on the interaction of each of the beverage consumer to the plurality of communication channels and based on the utilization of information by each of the beverage consumers.

Fig. 1

EP 3 745 410 A1

**Description**

The present invention relates to a method and software for monitoring beverage consumption of a human and for keeping hydration of a human in a physiological optimal range. Particularly, the invention relates to a self-learning method and software for monitoring hydration and beverage consumption of a human.
Water is primarily drunken by humans to satisfy thirst. Water is also drunken for other reasons such as accompanying a meal, refreshment and the like. Humans are increasingly demanding in selecting the suitable water.
After sports, when a human was sweating, he should drink water having a higher concentration of minerals. For accompanying a meal or for refreshment a human might prefer another type of water having a different and lower concentration of minerals. If hydration falls under a predetermined level a human may feel thirst or physiological deficiencies may occur.
Software for monitoring beverage consumption and hydration of a human may be implemented on dedicated devices or on a personal electronic device, such as a mobile telephone or tablet.

**Prior art**

WO 2016/090235 A1 discloses a portable hydration system including a mechanical or an electromechanical mechanism for dispensing additives into a liquid. Such additives include solids, liquids, powders, gases and include vitamins, minerals, nutritional supplements, pharmaceuticals and other consumables. Dispensing is initiated manually by direct human action, automatically by the device and/or external through an associated application on a human device. Dispensing is adjustable by context factors such as human preferences, location, activity and psychological status.
DE 20 2010 006 679 U1 discloses an apparatus for generating mineral water having a filter and at least one mineral container between the filter and the outlet. The apparatus further comprises a controller for controlling the feed of mineral from the at least one mineral container. If the water consumption by the human exceeds a daily limit of the daily water consumption feeding of minerals is stopped or another specific formulated water is dispensed.
WO 94/06547 A1 discloses a water purification and dispensing apparatus comprising a water inlet for obtaining water from a supply source, a water purification system for removing impurities from the source water and a mineral addition system for adding desired minerals into the purified water.
US 2013/0304265 A1 discloses a beverage dispenser having a transceiver to communicate with a bio sensor measuring a physiological parameter of a user. A controller is configured to alter a recipe of a beverage associated with the selection based on at least one of the data received from the bio sensor, the favorite beverage and the past beverage purchase such that a second recipe is formed.
This beverage dispenser has the disadvantage that the recipe is altered based on the physiological activity of the user after a significant time span and does not take into account the hydration of the human.

**Summary of the invention**

It is an object of the present invention to provide a computer implemented method for keeping a user in a physiological desired hydration range.
The object of the present invention is achieved by computer implemented method according to claim 1 or a computer implemented method according to claim 14. The depending claims relate to preferred embodiments.
The method of monitoring a beverage consumption of a human implemented by a computer comprises the steps of dividing a time span of a day into a plurality of time intervals and estimating the estimated hydration loss during each of the time intervals based on the activity of the human, the weight of the human, the temperature in the surroundings of the human and the humidity in the surroundings of the human. The method further comprises the step of evaluating for each of the time intervals the volume of beverage consumed by the human, and estimating for each of the time intervals an effective hydration loss of the human based on hydration lost and the beverage consumed by the human. The method further comprises the step of defining an euhydration threshold, depending on the weight of the user, wherein the euhydration threshold indicates that the euhydration of the human is at the lower limit of the euhydration. The volume of beverage consumed may be transmitted by a water dispenser to the method, such as the amount of beverage drawn by the user from the beverage dispenser. The volume of beverage consumed may be transmitted by a smart vessel having a sensor and a communication means, e.g. a smart bottle. The volume of beverage consumed from the smart vessel may be the beverage drunken from the smart vessel. The user may input the volume of beverage consumed on an input device, such as a touch sensitive display.
The hydration loss may be a relative fluid loss, a volume of fluid loss, a volume of hydration loss or an absolute fluid loss by sweating, breathing or the like.
The method defines an euhydration warning threshold depending on the weight of the user, wherein the euhydration warning threshold indicates an effective hydration loss of the human between average euhydration and the (lower)S euhydration threshold. The method sends the human a request to drink a first predetermined amount of beverage, when the effective hydration loss of the human within the at least one time interval exceeds the euhydration warning threshold.

The euhydration threshold indicates an effective hydration loss of the human at the lower limit of euhydration. Euhydration is the range, in which the human has the optimal hydration from a physiological or medical aspect. Since the method according to the present invention warns the user before hydration is lower than euhydration and requests the user to drink water before the actual hydration is lower than the euhydration, the method can ensure that the human is kept in the range of ideal hydration (euhydration).

Further, the inventive method divides the time span of a day into comparably small intervals. For example, each time interval may be a duration of one hour. Thereby, the human is monitored within comparably short time intervals. Further, the inventive method ensures, that the human is warned at an early stage that the actual hydration of the human might escape the euhydration range. Thereby, the human (user) is generally only requested to drink small amounts of beverage.

The estimated hydration loss (HL) [I], particularly the fluid loss, may be estimated by the following formula as a function of activity [MET (kcal/h)], weight [kg], temperature [C], humidity [%]:

$$\text{HL per hour} = (\text{activity} * \text{weight} * \text{temperature} + \text{humidity}^2)/1450 * 0.029;$$

The estimated hydration loss per day is:)

$$HL\ per\ day = \sum_{i=awake\ time}^{sleep\ time} \frac{\text{HL}}{\text{h}} i$$

The first predetermined amount of beverage to be drunken by the human to balance the hydration loss may range between 80% to approximately 120%, preferably between 90% to 110% of the effective hydration loss.

The time span may commence at the time of getting up of the human and may end with the bedtime of the human. The method assumes that the human is euhydrated at the time of getting up. The human cannot drink beverage during bedtime. Therefore, the user is not monitored during the night.

The method defines a thirst threshold depending on the weight of the human, wherein the first threshold indicates a hydration of the human, when the human starts getting thirsty and feeling thirst. The method may send the human a request to drink water, when the effective hydration loss of the human exceeds the thirst threshold.

The method may define a balance threshold depending on the weight of the human, wherein the balance threshold indicates a hydration of the human, when the human starts getting out of mental balance and/or physical balance. The method may send the human a request to drink water, when the hydration of the human exceeds the balance threshold.

The euhydration warning threshold may range between approximately 0.05% to approximately 0.14% of the body weight of the human. The (upper and lower) euhydration threshold may range between approximately 0.15% to approximately 0.24% of the body weight of the human. The thirst threshold may arrange between approximately 0.35% to approximately 0.64%, preferably between approximately 0.45% to approximately 0.54% of the body weight of the human. The balance threshold may range between approximately 0.85% to approximately 1.14%, preferably between approximately 0.95% to approximately 1.04% of the body weight of the human. The deficiency threshold may range between approximately 1.85% to approximately 2.14%, preferably between approximately 1.95% to approximately 2.04% of the body weight of the human. The euhydration warning threshold, lower euhydration threshold, the thirst threshold, the balance threshold and the deficiency threshold may have a negative sign, while the upper euhydration threshold may have a positive sign.

The method may assign the human a euhydration state, if hydration of the human did not exceed the euhydration threshold, and the method may assign the human an intermediate state, if the hydration of the human exceeded the euhydration threshold, and did not exceed the thirst threshold. The method may assign the human a thirst state, if the hydration of the human exceeded the thirst threshold, and did not exceed the deficiency threshold. The method may assign the human an off-balance state, if hydration of the human is below the balance threshold.

The method may determine a hydration balance score based on how long the hydration of the human is in the euhydration state, the intermediate state, the thirst state, and the off-balance state. The method may determine a hydration volume score based on the sum of the effective hydration loss of all time intervals of one day. The method may determine a hydration score based on the hydration balance score and the hydration volume score and display the hydration score to the human. The hydration score indicates, whether the human is hydrated according to his physiological and medical requirements.

The method further comprises the step of defining a hydration balance goal or requesting a user to input the hydration balance goal, wherein the hydration balance goal defines the hydration balance score to be achieved by the human. The method may determine the hydration balance score achieved by the human. The method may request the user to adapt the hydration balance goal based on the achieved hydration balance score, if the achieved hydration balance score is lower than the hydration balance goal for a first predetermined time span. The software implemented method learns (machine learning) that the user cannot achieve the hydration balance goal currently. Therefore, the user is requested to input an amended hydration balance goal that can be more realistically achieved by him. Thus, the user continues to user the computer implemented method and does not discontinue using the computer implemented method due to a hydration balance goal that cannot be achieved by him.

The method may increase the hydration goal based on the hydration score. The hydration score may be monitored over a plurality of days. The final goal is to keep the human as long as possible in the euhydration state, and to keep the human to drink the recommended amount of beverage for ensuring proper hydration of the human. If the user does not drink the requested amount of beverage, the hydration balance goal may be reduced to a lower level that can be easier achieved by the human. If the hydration balance goal may be easier achieved by the human, the human continues to use the software implementing the method according to the present invention and proper hydration of the human can be ensured.

The method may further define a hydration volume goal, wherein the hydration volume goal defines the hydration volume score to be achieved by the human. The method may determine the hydration balance score and hydration volume score achieved by the human. The method may request the user to adapt the hydration balance goal, if the achieved hydration volume score is lower than the hydration volume goal for the first predetermined time span.

In one embodiment, the method may request a beverage consumption motivation from the human, wherein the beverage consumption motivation comprises at least a first category of beverage consumption motivations, wherein the first category of beverage consumption motivations comprises at least one of wellness, fitness, vitality and concentration. The method may request the human to drink beverage, if the difference between the hydration volume goal and the hydration volume score is larger than a second fulfillment threshold. The method assigns the human a hydration balance goal based on the beverage consumption motivation selected from the first category of beverage consumption motivations. In one embodiment the method may adapt the hydration volume goal based on the achieved hydration volume score, if the achieved hydration volume score is lower than the hydration volume goal for a second predetermined time span. The method may also monitor the total volume score to determine the goal fulfillment by the following formula:

$$\text{Daily total volume score} = \frac{\sum_{i=awake\,time}^{sleep\,time} beverage\ consumed\ per\ timer\ interval * i}{HL\ per\ day};$$

The beverage consumption motivation comprises a second category of beverage consumption motivations, wherein the second category of beverage consumption motivations comprises at least one of health and weight loss. The method may also comprise the step of assigning the human the hydration balance goal and a hydration volume goal based on the beverage consumption motivation selected from the second category of beverage consumption motivations.

The method may request the human to input an activity level, such as by requesting the user to enter his personally preferred activity level. The method may estimate the estimated hydration loss based on the input activity level. The method may also measure the actual activity level by a sensor or an app of a personal electronic device (health app), for example by a sensor or an app running on a personal electronic device (heath app, fitness app). If the actual activity differs from the activity goal for a predetermined level difference, the human is requested to adapt the activity goal.

The method may estimate the activity of the user by importing data from the calendar and by recording regular physical action that is repeated on regular basis, such as weekly visits of fitness studios.

The method may update the estimated hydration loss based on the actual activity. In a first step the hydration loss due to activity is estimated based on an activity level input by a user. In the second step the estimated hydration loss is refined by the actual activity of the user.

The invention also discloses a method for defining types of beverage consumers implemented by a computer. The method assesses the hydration development of a plurality of beverage consumers by monitoring the volume of beverage consumed by each of the beverage consumers in at least one time interval and the hydration loss of each of the beverage consumers within a plurality of time intervals of a predetermined time range comprising a plurality of days. The hydration development may be assessed by the method of monitoring a beverage consumption of a human implemented by a computer described above and claimed in claims 1 to 13.

The method assesses the physical conditions and/or location of each of the beverage consumers by assessing at least the physical activity of each of the beverage consumers and the weather in the environment of each of the beverage consumers.

The method sends a plurality of messages of a plurality of types to each of the beverage consumers by at least one communication means. The method assesses the interaction of each of the beverage consumers to the communication means. This may be embodied by a self-learning or machine learning method. The communication means may include email, messenger messages, SMS or push notifications of a software running on a personal electronic device, such as a smart phone or tablet computer. The method may assess the time until the user opens a message. The method may assess the utilization of information transferred by each of the plurality of messages by each of the beverage consumers. This may also be embodied by a self-learning or machine learning method. The method may assess, whether the user reads messages or information provided by the method and whether the user changes his beverage consumption based on the messages sent.

The method may define a plurality of beverage consumer clusters based on the hydration development of each of the beverage consumers, based on the physical conditions and/or location of each of the beverage consumer, based on the interaction of the beverage consumer to the plurality of communication channels and based on the utilization of information by each of the beverage consumers.

The above method defines different types of users based on their behavior and hydration development. This clustering of users may support the method in developing a hydration strategy for the different types of users for optimizing their hydration level. The method may determine groups of beverage consumers in different cultures for adapting drinking recommendations to the different cultures.

The step of assessing the hydration development of the plurality of beverage consumer may be performed by the method of monitoring a beverage consumption of a human described above.

The method for defining types of beverage consumers may include the step of assessing for a plurality of beverage consumers the influence of a physical condition and/or location to the hydration development and storing the influence of a physical condition and/or location to the hydration development for a plurality of humans as a first classification.

The method may comprise the step of assessing for a plurality of beverage consumers the dependency of the utilization of information on the type of messages and/or communication means and store the dependency of utilization of information on the type of message and/or communication means as a second classification.

The method may assess the hydration development of a single beverage consumer by monitoring the volume of beverage consumed by each of the beverage consumers in at least one time interval and the hydration loss of each of the beverage consumers within a plurality of time intervals of a predetermined time range comprising a plurality of days. The method may assess the physical conditions and/or location of a single beverage consumer by accessing at least the physical activity of the beverage consumers and the weather in the environment of the beverage consumer. The method may determine the influence of a physical condition and/or location to the hydration development by reading from the first classification. The method may output a beverage consumption suggestion depending on the hydration development and the influence of a physical condition and/or location on the hydration development read from the first classification. These steps may be implemented by self-learning and/or recursive learning.

The method may determine the dependency of the utilization of information on the type of message and/or communication means by reading from the second classification and outputting the beverage consumption suggestion by the type of message having the best utilization of information, e.g for the respective user and/or user group.

The above described method of monitoring a beverage consumption of a human may be implemented by a computer. Therefore, the present invention discloses a computer program product that when loaded into a memory of a computer comprising a processor executes the above defined steps of monitoring a beverage consumption of a human as claimed in claims 1 to 13. This method may be implemented by a personal electronic device, such as a mobile phone or a tablet computer. The method may be implemented by a so-called app.

The method of defining types of beverage consumers may be also implemented by a computer. Therefore, the present invention discloses a computer program product that when loaded into a memory of a computer comprising a processor executes the above defined steps of the method for defining the types of beverage consumers as claimed in claims 14 to 19.

The beverage may be water. The beverage may be water individually mineralized, tempered and carbonized by a water dispenser according to the preference of a user.

## Short description of the drawings

The invention is now described in further detail with reference to the attached drawings showing non-limiting examples of the present invention, wherein

Figure 1 depicts hydration loss of a human during daytime without consuming beverage;

Figures 2 to 5 depict hydration of a human regularly consuming beverage;

Figure 6 shows an embodiment of the inventive method for clustering behavior data, context data, interaction data and feedback data;

Figure 7 shows an embodiment of the inventive method for classification of relevant contexts;

Figure 8 shows an embodiment of the present invention for classifying beverage consumption interactions; and

Figure 9 shows an embodiment of the method according to the present invention for refining beverage consumption suggestion based on the context.

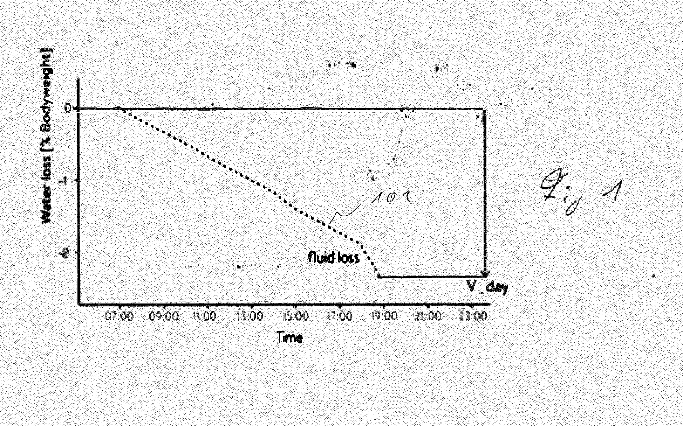

Reference is made to figure 1 showing the hydration loss of a human during the day, if the human does not consume any beverage. The hydration loss (HL) and fluid loss [l], respectively can be estimated by the following formula as a function of activity [MET (kcal/h)], weight [kg], temperature [C], humidity [%]:

$$HL \text{ per hour} = (activity * weight * temperature+humidity^2)/1450 * 0.029;$$

The estimated hydration loss per day is:

$$HL \text{ per day} = \sum_{i=awake\ time}^{sleep\ time} \frac{HL}{h}i$$

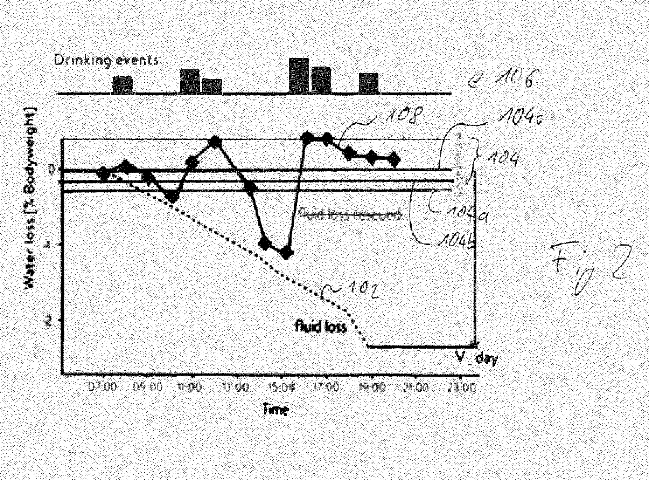

Reference is now made to figure 2. The time span in which a user is awake, generally from getting up until bedtime is divided into a plurality of intervals, such as intervals of one hour. The inventive estimates by the above formula the estimated hydration loss 102 at the end of each time interval. Further, the inventive method monitors drinking events 106, at which the human monitored drinks beverage. The method monitors the volume of beverage consumed by the user during each time interval. Thereafter, the method calculates the effective hydration loss 108 per time interval. The volume of beverage consumed may be transmitted by a water dispenser to the method, such as the amount of beverage drawn by the user from the beverage dispenser. The volume of beverage consumed may be transmitted by a smart vessel having a sensor and a communication means, e.g. a smart bottle. The volume of beverage consumed from the smart vessel may be the beverage drunken from the smart vessel. The inventive method may be implemented by a software running on a computer, an app running on personal electronic device, such as a smart phone or a tablet computer, or the like.

The inventive method tries to keep the user in the range of euhydration 104. The lower limit of euhydration 104a is a fluid loss of approximately 0.2% of the body weight of the human user. For preventing the effective hydration loss of the human to be larger than the threshold of euhydration 104a the user is notified by a message, if the effective hydration loss exceeds a euhydration warning threshold 104b. In one embodiment, the euhydration warning threshold may be 0.1% of the body weight of the human user.

In case the inventive method determines that the user has been drinking more than approximately 500 ml to approximately 800 ml per hour, the inventive method outputs a warning to the user that only approximately 500 ml to approximately 800 ml per hour can be reabsorbed by the human body.

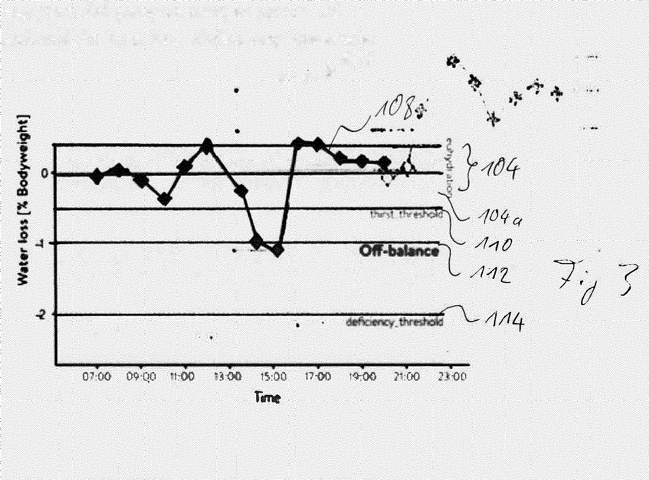

Reference is made to figure 3 showing further important thresholds, namely a thirst threshold 110, an off-balance threshold 112 and a deficiency threshold 114. Generally, humans feel thirst at a fluid loss of approximately 0.5% of the body weight. This defines the thirst threshold 110. The inventive method transmits a message to the human user, if the effective hydration loss 108 exceeds the thirst threshold 110.

If the user doesn't drink beverage after the thirst threshold warning message, the effective hydration loss of the user may surpass 1 % of the body weight of the user. This threshold is called off-balance, since the user does not feel comfortable any more. The inventive method sends the human user an off-balance warning message, as soon as the effective hydration loss 108 surpasses the off-balance threshold 112.

If the user doesn't drink beverage, the effective hydration loss 108 may further increase and surpass the deficiency threshold 114. If the effective hydration loss 108 surpasses the deficiency threshold 114, a user may experience physical and cognitive deficiencies. Generally, the deficiency threshold is approximately 2% of the body weight of the human user.

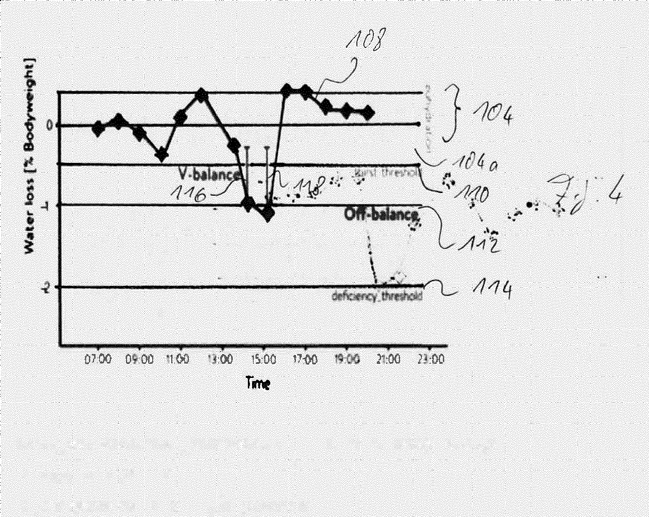

Reference is made to figure 4 showing a strategy for rehydrating the human user. The user is in the status of euhydration until approximately 2:00 p.m. Since the user has surpassed the euhydration threshold 104a, the thirst threshold 110 and the off-balance threshold 112, the user is notified to drink a certain amount of beverage. Generally, the inventive method requests the user to drink the volume of water or other beverage comprising water corresponding to the volume necessary to bring the user into euhydration. However, the amount necessary for bringing the user back into euhydration may exceed an amount that can be resorbed by the human body.

With reference to figure 4, the method recommended the user at 2:00 p.m. to drink a first beverage amount 116 by a message. Obviously, the user didn't follow the recommendation transmitted by the message. Therefore, the body continues to be dehydrated until 3:00 p.m. At 3:00 p.m. the user is notified to drink the second beverage amount 118. Since the user follows the recommendation of the method, he returns in the state of euhydration at 4:00 p.m.

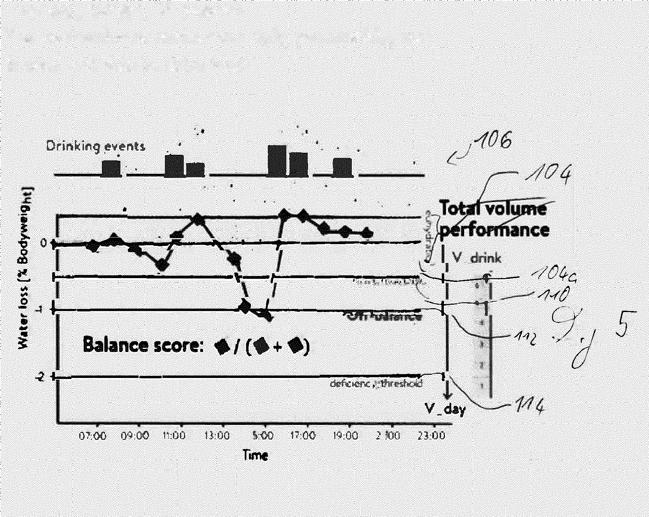

Reference is made to figure 5 showing a scoring of the beverage consumption performance of the human user. The method calculates the total volume of beverage consumed by the user as the sum of the volume of beverage consumed by the human during each of the time intervals. The method also calculates a hydration balance score according to the following formula:

$$\text{Hydration balance score} = \text{hours in balance/total hours awake;}$$

The method also calculates a daily total volume score by the following formula:

$$\text{Daily total volume score} = \frac{\sum_{i=awake\ time}^{sleep\ time} beverage\ consumed\ per\ timer\ interval * i}{HL\ per\ day}.$$

The above method may be executed by a software (app) running on a personal electronic device, such as a smart phone or tablet computer.

The method may assign the human a euhydration state, if hydration of the human did not exceed the euhydration threshold, and the method may assign the human an intermediate state, if the hydration of the human exceeded the euhydration threshold, and did not exceed the thirst threshold. The method may assign the human a thirst state, if the hydration of the human exceeded the thirst threshold, and did not exceed the deficiency threshold. The method may assign the human and off-balance state, if hydration of the human is below the balance threshold.

The method may determine a hydration balance score based on how long the hydration of the human is in the euhydration state, the intermediate state, the thirst state, and the off-balance state. The method may determine a hydration volume score based on the sum of the effective hydration loss of all time intervals of one day. The method may determine a hydration score based on the hydration balance score and the hydration volume score and display the hydration score to the human. The hydration score indicates whether the human is hydrated according to his physiological and medical requirements.

The method further comprises the step of defining a hydration balance goal or requesting a user to input the hydration balance goal, wherein the hydration balance goal defines the hydration balance score to be achieved by the human. The method may determine the hydration balance score achieved by the human. The method may request the user to adapt the hydration balance goal based on the achieved hydration balance score, if the achieved hydration balance score is lower than the hydration balance goal for a first predetermined time span. The software implemented method learns (machine learning) that the user cannot achieve the hydration balance goal currently. Therefore, the user is requested to input an amended hydration balance goal that can be more realistically achieved by him. Thus, the user continues to user the computer implemented method and does not discontinue using the computer implemented method due to a hydration balance goal that cannot be achieved by him.

The method may increase the hydration goal based on the achieved hydration score during a predetermined time span. The hydration score may be monitored over a plurality of days. The final goal is to keep the human as long as possible in the euhydration state, and to keep the human to drink the recommended amount of beverage for ensuring proper hydration of the human. If the user does not drink the requested amount of beverage, the hydration balance goal may be reduced to a lower level that can be easier achieved by the human. If the hydration balance goal may be easier achieved by the human, the human continues to use the software implementing the method according to the present invention and proper hydration of the human can be ensured.

The method may further define a hydration volume goal, wherein the hydration volume goal defines the hydration volume score to be achieved by the human. The method may determine the hydration balance score and hydration volume score achieved by the human. The method may request the user to adapt the hydration balance goal, if the achieved hydration volume score is lower than the hydration volume goal for the first predetermined time span.

In one embodiment, the method may request a beverage consumption motivation from the human, wherein the beverage consumption motivation comprises at least a first category of beverage consumption motivations, wherein the first category of beverage consumption motivations comprises at least one of wellness, fitness, vitality and concentration. The method may request the human to drink beverage, if the difference between the hydration volume goal and the hydration volume score is larger than a second fulfillment threshold. The method assigns the human a hydration balance goal based on the beverage consumption motivation selected from the first category of beverage consumption motivations. In one embodiment the method may adapt the hydration volume goal based on the achieved hydration volume score, if the achieved hydration volume score is lower than the hydration volume goal for a second predetermined time span. The method may also monitor the total volume score to determine the goal fulfillment by the following formula:

$$\text{Daily total volume score} = \frac{\sum_{i=awake\ time}^{sleep\ time} beverage\ consumed\ per\ timer\ interval * i}{HL\ per\ day}\ ;$$

The beverage consumption motivation comprises a second category of beverage consumption motivations, wherein the second category of beverage consumption motivations comprises at least one of health and weight loss. The method may also comprise the step of assigning the human the hydration balance goal and a hydration volume goal based on the beverage consumption motivation selected from the second category of beverage consumption motivations.

The method may request the human to input an activity level, such as by requesting the user to enter his personally preferred activity level. The method may estimate the estimated hydration loss based on the input activity level. The method may also measure the actual activity level by a sensor or an app of a personal electronic device (health app), for example by a sensor or an app running on a personal electronic device (heath app, fitness app). If the actual activity differs from the activity goal for a predetermined level difference, the human is requested to adapt the activity goal.

The method may estimate the activity of the user by importing data from the calendar and by recording regular physical action that is repeated on regular basis, such as weekly visits of fitness studios.

The method may update the estimated hydration loss based on the actual activity. In a first step the hydration loss due to activity is estimated based on an activity level input by a user. In the second step the estimated hydration loss is refined by the actual activity of the user.

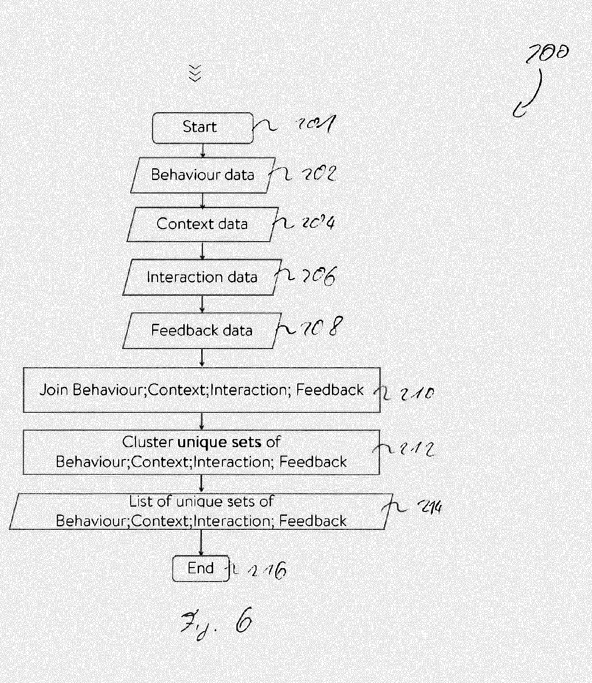

Reference is made to figure 6 showing a general flowchart of the method of the present invention, particularly a method for defining types of beverage consumers. The method may be implemented on a mobile device, such as a smart phone and partially on a backend computer. The method clusters users into groups and automatically identifies recommendations for hydrating. The method commences in step 200. In step 202, the method collects behavior data. The behavior data reflects the drinking behavior of a user, evaluated by the volume and time stamps of water consumption. Particularly, the behavior data includes assessing the hydration development of a plurality of beverage consumers by monitoring the volume of beverage consumed by each of the beverage consumer in at least one time interval and the hydration loss of each of the beverage consumers within a plurality of time intervals of a predetermined time range comprising a plurality of days. In step 204 the method collects context data including a behavior reflecting activity of the user, the weather in the environment of the user and location data of the user. Particularly, the method assesses the physical conditions and/or location of each of the beverage consumers by at least the physical activity of each of the beverage consumers and the weather in the environment of each of the beverage consumers.

In step 206, the method collects interaction data comprising both type of user interaction via the inventive method, such as notifications of a software (app) running on a mobile device, email, web notifications, app push notifications as well as the frequency of these interactions. Particularly, the method sends a plurality of messages of a plurality of types to each of the beverage consumers by at least one communication means. Further, the method assesses the interaction of each of the beverage consumers to the plurality of communication means including for example app push notifications, web notifications, email, SMS, messenger notifications or the like.

In step 208 the method according to the present invention collects feedback data, which are measurements of user reaction to the interactions, such as clicking on a notification, following and reading send links, or the like.

In step 210 the method according to the present invention joins behavior data, context data, interaction data and feedback data.

In step 212 the inventive method clusters sets of behavior data, context data, interaction data and feedback data. The clustered sets can be found by dimension reduction and clustering of the features within the behavior data, context data, interaction data and feedback data, for example by a principal component analysis. Particularly, the method defines a plurality of beverage consumer clusters based on the hydration development of each of the beverage consumer, based on the physical conditions and/or location of each of the beverage consumer, based on the interaction of each of the beverage consumers to the plurality of communication channels and based on the utilization information by each of the beverage consumers.

Clustering a plurality of beverage consumers allows the method to identify similar users and also to optimize drinking recommendations to the different user types and/or user clusters.

In order to join data coming from the different sources a first preprocessing step of normalization and standardization is to be performed to compare numeric values obtained from different scales (e.g. activity in kcal vs water consumption in ml/h). A standard min-max normalization step will rescale all feature values to a range in [0, 1]. With a following zero-mean standardization would further rescale each distribution of values so that the mean of observed values is 0 and the standard deviation is 1.

The method can create with the normalized and standardized vectors of features a multidimensional array of observations (users) and variables (interaction, behavior, context and feedback measures) to find clusters of users with similar patterns. To cluster the method may first carry out a dimensionality reduction step with a Principal Component Analysis (PCA) and on that variance space the method may cluster with a hierarchical clustering implementation. Upon separating clusters of users according to their features patters the method can label these user groups for steps described below.

In step 214 the method stores the list unique sets of behavior data, context data, interaction data and feedback data in a database. This part of the method terminates at step 216.

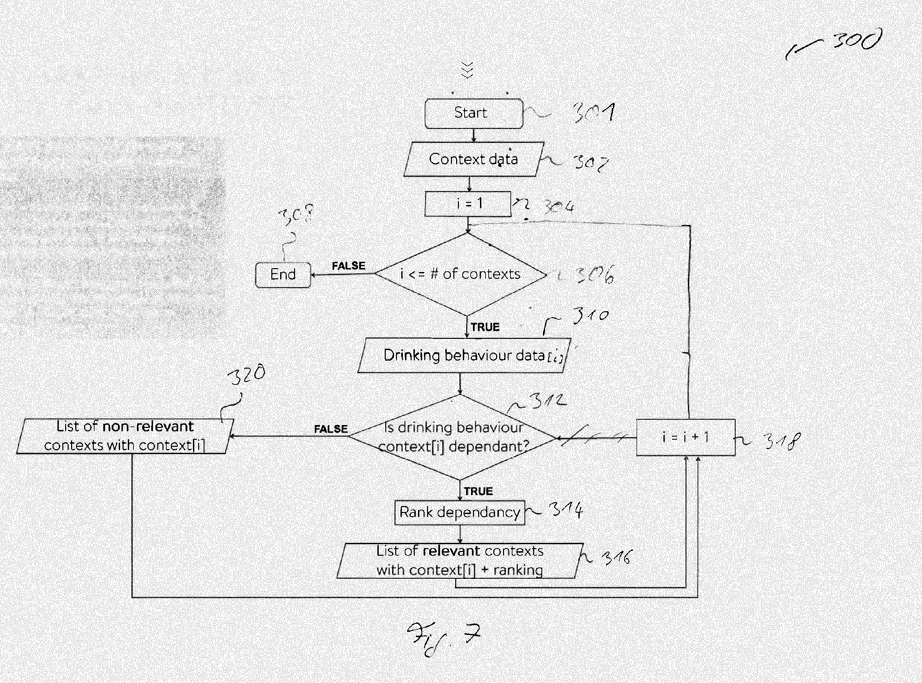

Reference is made to figure 7 showing the method steps for classifying of relevant contexts. The context dependency defines a dependency of the behavior data of the context data, if the drinking behavior deviates from a base line (standard behavior) of a drinking behavior of a user group. A ranking dependency may be calculated by comparing the magnitude of deviation from the baseline for all behavior dependent contexts. In other words, context data is considered to be relevant, if a significant amount of users change their beverage consumption behavior based on the context and/or location.

The method commences in step 301 and reads in step 302 the context data stored in a database according to the method of clustering data 200. The method 200 has stored the list of unique sets of behavior data, context data, interaction data and feedback data in step 214 in a database.

In step 304 index i is increased by 1. In step 306, the method determines, whether the index i is smaller or equal to the number of stored contexts in the context data. If the index i is smaller or equal and the number of contexts, the method proceeds to step 310 and loads the drinking behavior data from the database as stored in step 214.

In step 312, the method determines, whether the drinking behavior data depends on the context. In other words, the method determines based on the stored behavior data and context data, if the behavior of a plurality of user changes depending on the context. For example, a first plurality of users may drink more water, when flying by an airplane. Another group of user might drink more water when flying in an airplane. Another group of user may drink more water before or during physical exercise, while another group of users may drink more water after the physical exercise.

If the method determines in step 312 a dependency of the drinking behavior on the context, the degree of dependency is ranked. This can be achieved by determining how much a user, a plurality of users and/or a user group changes its beverage consumption behavior based on a particular context. In step 316 a list of all relevant contexts depending on the index i and the ranking is stored.

If the method determines in step 312 that a particular user, a group of users and/or a plurality of users does not change its beverage consumption behavior for a particular context, this context is stored in the list of non-relevant contexts depending on the index i.

The method continues from step 316 and 320 to step 318 and increases the index i by 1. Thereafter the method continues to step 306 and continues to proceed with step 310, until the index i is larger than the number of contexts, in which case the method terminates by proceeding to step 308.

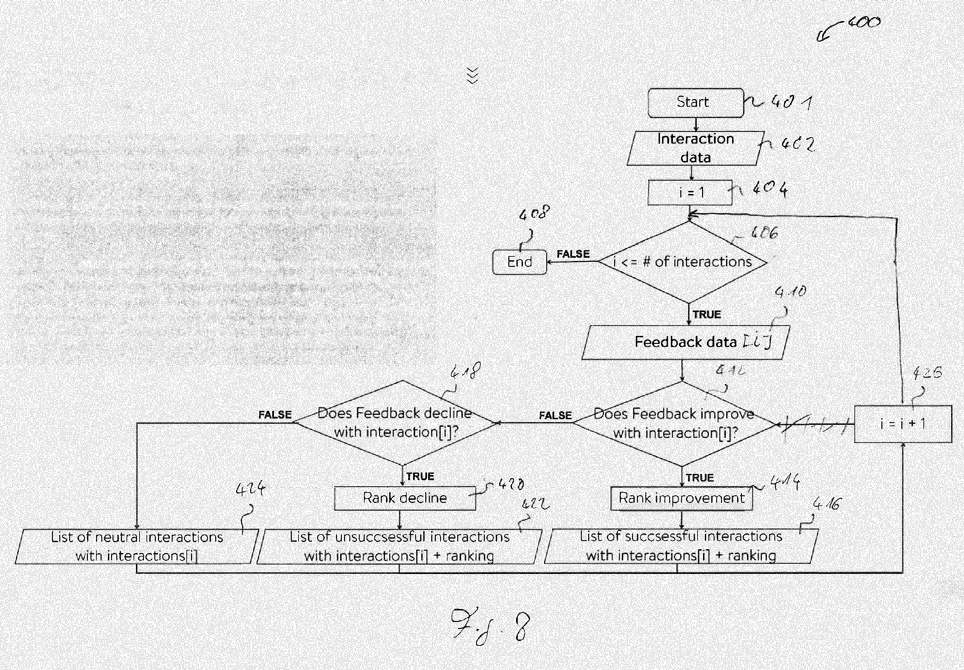

Reference is made to figure 8 showing an embodiment of the inventive method 400 for classifying interactions on beverage consumption suggestions. The method starts at step 401 and loads interaction data stored in step 214 from a database in step 402. The method continues with step 404 and sets an index i to 1.

The method continues with step 406 and verifies, whether the index i is smaller than the number of interaction data sets loaded in step 402. If the index i is smaller or equal than the number of interaction sets, the method continues to step 410 and loads feedback data stored in a database in step 214.

As described above, the interaction data generally comprises both the type of user interaction with the method, such as an app, a web notification, an app push notification as well as the frequency of these interactions. Feedback data generally includes measurements of user reactions to interactions, such as clicking on a notification, following and reading sent links, water consumption entries or the like.

The method proceeds with step 412 and verifies, whether the feedback improves with the interaction i. If the feedback improves with interaction i, the method continues with step 414.

In one example for improved feedback with the interaction i, the user has been recording water consumption for two weeks and initially managed to keep in balance only in the mornings and in the evenings. As interaction, an app notification is sent to the user half an hour after lunch time at the beginning and the end of the week reminding them to drink a glass of water to improve digestion. The feedback (reaction) of the user starts consuming water also in the afternoon, increasing the total time they are spent in balance.

In step 414 the method evaluates, whether the ranking of the feedback improved depending on the interaction. A baseline of normal feedback is defined by the expected reaction to the interaction, such as by clicking a push notification. A baseline is defined by the average or normal feedback of a user group. Feedback improvement may be measured in terms of speed of reaction, as well as added reactions to a particular interaction, such as a user clicks a push notification and regularly consumes beverage over a predetermined time span.

The method continues with step 416 and stores the interaction i and the ranking thereof in the list of successful interactions. Then, the method continues with step 426.

If the method determines in step 412 that the feedback does not improve with interaction i, the method continues with step 418. In step 418, the method determines whether feedback declines with interaction i. Feedback decline can be measured in terms of the expected reaction. In other words, the interaction i is smaller than the average reaction of a user, a user group and/or a user cluster. If the feedback for interaction i has declined, the method continues with step 420 and declines the ranking of interaction i. Then, the method continues with step 422 and inserts the interaction i and the ranking in the list of unsuccessful interactions before continuing with step 426.

If the method determines in step 418 that feedback of interaction i did not decline, the method continues with step 424 and stores interaction i in the list of neutral interactions and continues with step 426.

In one example for feedback decline with the interaction i, the user has been recording water consumption for two weeks and initially managed to keep in balance only in the mornings and in the evenings. As interaction, a push notification is sent to the user every day showing them their statistics to help them get motivated to reach their goals. The feedback (reaction) of the user is that the user gets annoyed with so many notifications and disables the communication with the app.

In one example for neutral feedback with the interaction i, the user has been recording water consumption for two weeks and initially managed to keep in balance only in the mornings and in the evenings. An email is sent to the user showing them their statistics and making clear that water consumption in the afternoon is missing as interaction. The feedback (reaction) is that the user ignores the email and doesn't change their behavior.

In step 426 the index i is increased by 1 and the method continues with step 406. In step 406 the method determines, whether the index i is smaller or equal than the number of interactions. If the index i is smaller or equal than the number of interactions, the method continues with step 410, as described above. In the alternative, the method continues with step 408 and ends.

With a labeled group of users according to different feature combinations, the method can train a machine learning model to identify the patterns of context-interaction-feedback data previously defined as successful. As a first step the method trains a standard machine learning model e.g. random forest, an ensemble learning method for classification based on training a multitude of decision trees to classify interactions. During a second step, the method trains a neuronal network to perform the same task with more efficiency.

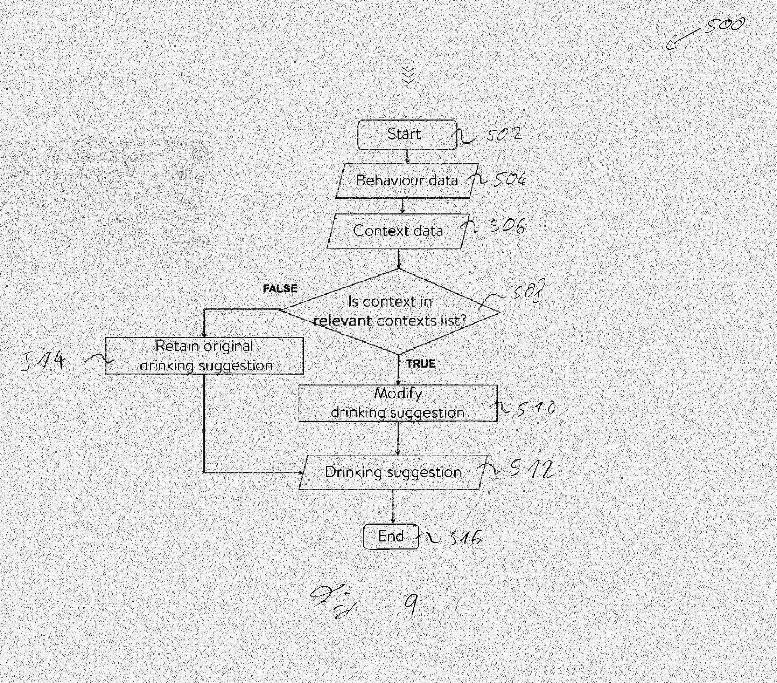

In figure 9, another embodiment of the inventive method 500 is depicted. The method starts with step 502 and continues with steps 504 and 506, in which behavior data and context data is loaded from the database is stored in step 214. As mentioned above, behavior data reflects the user drinking behavior, as may be evaluated by the amount and timing based on beverage consumption recommendation. Context data includes activity of the user, whether in the environment of the user and location data.

The embodiment of the method shown in figure 9 refines a beverage consumption suggestion based on the context. For refining the beverage consumption suggestion based on the context, the method determines in step 508, whether the context is in the relevant context list as determined by the steps of classification of relevant context 300 shown in figure 7. The list of relevant context and the ranking is stored in step 316 in a database.

If the context is in the list of relevant contexts, the method continues with step 510 and modifies the drinking suggestion. For example, if the method knows based on the digital calendar of a user that he will commence physical activity, enter an aircraft, is at a location with low humidity, is at a location with high temperature or the like, the method may recommend the user to consume beverage before he enters such location or commences physical activity or during physical activity or during the time spent at the before mentioned locations.

In step 512 the beverage consumption suggestion is output.

If the method determines in step 508 that the context is not in the relevant context list, the method continues with 114 and retains the original drinking suggestion, which is output also in step 512. After step 512, the method continues to step 516 and the embodiment of the method 500 according to figure 9 ends.

The computer implemented method monitors hydration of a human user such that the human user is in euhydration as long as possible. The method also classifies types of users to support them by suitable recommendations to keep themselves in euhydration as long as possible. The method is a self-learning method. The beverage can be water.

**Claims**

1. A method of monitoring a beverage consumption of a human implemented by a computer, comprising the following steps:

   - dividing a timespan of a day into a plurality of time intervals;
   - estimating the estimated hydration loss during each of the time intervals based on the activity of the human, the weight of the human, the temperature in the surrounding of the human and the humidity in the surrounding of the human;
   - evaluating for each of the time intervals the volume of beverage consumed by the human;
   - estimating for each of the time intervals an effective hydration loss of the human based on the estimated hydration loss and the volume of beverage consumed by the human;
   - defining an euhydration threshold depending on the weight of the human, wherein the euhydration threshold indicates that the hydration of the human is at the lower limit of euhydration;
   - defining an euhydration warning threshold depending on the weight of the human, wherein the euhydration warning threshold indicates an effective hydration loss of the human between average euhydration and the euhydration threshold; and
   - sending the human a request to drink a first predetermined volume of beverage, when the effective hydration loss of the human within the at least one time interval exceeds the euhydration warning threshold.

2. The method according to claim 1, wherein the first predetermined volume of beverage ranges between 80% to approximately 120 %, preferably between 90 % to 110 % of the effective hydration loss.

3. The method according to claim 1 or 2, wherein the timespan commences at the time of getting up of the human and ends with bedtime of the human.

4. The method according to any one of claims 1 to 3, further comprising at least one of the following steps:

   - defining a thirst threshold depending on the weight of the human, wherein the thirst threshold indicates a hydration of the human, when the human starts getting thirst;
   - sending the human a request to drink water when effective hydration loss of the human exceeds the thirst threshold.

5. The method according to claim 1 or 4, further comprising the following steps:

   - defining a balance threshold depending on the weight of the human, wherein the balance threshold indicates a hydration of the human, when the human starts getting out of mental and/or physical balance;
   - sending the human a request to drink water, when the hydration of the human within exceeds the balance threshold.

6. The method according to any one of claims 1 to 5, **characterized by** at least one of the following:

   - the euhydration warning threshold ranges between approximately 0,05 % to approximately 0,14 % of the body weight of the human;
   - the euhydration threshold ranges between approximately 0,15 % to approximately 0,24 % of the body weight of the human;
   - the thirst threshold ranges between approximately 0,35 % to approximately 0,64 %, preferably between approximately 0,45 % to approximately 0,54 % of the body weight of the human;
   - the deficiency threshold ranges between approximately 0,85 % to approximately 1,14 %, preferably between approximately 0,95 % to approximately 1,04 % of the body weight of the human.

7. The method according to any one of claims 1 to 6, further comprising the following steps:

   - assigning the human a euhydration state, if the hydration of the human did not exceed the euhydration threshold;
   - assigning the human an intermediate state, if the hydration of the human exceeded the euhydration threshold

and did not exceed the thirst threshold;
- assigning the human a thirst state, if the hydration of the human exceeded the thirst threshold and did not exceed the deficiency threshold;
- assigning the human an off-balance state, if the hydration of the human is below the balance threshold;
- determining a hydration balance score based on how long the hydration of the human is in the euhydration state, the intermediate state, the thirst state and the off-balance state; and
- determining a hydration score based on the hydration balance score and the hydration volume score; and
- displaying the hydration score to the human.

8. The method according to claim 7, further comprising the following steps:

- defining or requesting the user to enter a hydration balance goal, wherein hydration balance goal defines the hydration balance score to be achieved by the human;
- determining the hydration balance score achieved by the human; and
- requesting a user to adapt the hydration balance goal, if the achieved hydration balance score is lower than the hydration balance goal for a first predetermined time span.

9. Method according to claim 8, further comprising the following steps:

- determining a hydration volume score based on sum of the effective hydration loss of all time intervals of one day;
- defining a hydration volume goal, wherein the hydration volume goal defines the hydration volume score to be achieved by the human;
- determining the hydration volume score achieved by the human; and
- requesting a user to adapt the hydration volume goal, if the achieved hydration volume score is lower than the hydration volume goal for the first predetermined time span.

10. The method according to any one of claims 7 to 9, comprising the following steps:

- requesting a beverage consumption motivation from the human, wherein the beverage consumption motivation comprises at least a first category of beverage consumption motivations, wherein the first category of beverage consumption motivations comprises at least one of wellness, fitness, vitality and concentration; and
- assigning the human a hydration balance goal based on the beverage consumption motivation selected from the first category of beverage consumption motivations.

11. The method according to any one of claims 7 to 10, wherein

- the beverage consumption motivation comprises a second category of beverage consumption motivations, wherein the second category of beverage consumption motivations comprises at least one of health and weight loss;
further comprising the step of
- assigning the human a hydration balance goal and a hydration volume goal based on the beverage consumption motivation selected from the second category of beverage consumption motivations.

12. The method according to claim 9 or 10, further comprising the following steps:

- requesting the human to input an activity level;
- estimating the estimated hydration loss based on the input activity level;
- measuring the actual activity of the human;
- if the actual activity differs from the input activity level for a predetermined level difference, requesting the human to adapt the input activity level.

13. Method according to claim 12, updating the estimated hydration loss based on the actual activity.

14. A method for defining types of beverage consumers implemented by a computer, comprising the following steps:

- assessing the hydration development of a plurality of beverage consumers by monitoring the volume of beverage consumed by each of the beverage consumers in at least one time interval and the hydration loss of each of the beverage consumers within a plurality of time intervals of a predetermined time range comprising a plurality of days;

- assessing the physical conditions and/or location of each of the beverage consumers by assessing at least the physical activity of each of the beverage consumers and the weather in the environment of each of the beverage consumers;
- sending a plurality of messages of a plurality of types to each of the beverage consumers by at least one communication means;
- assessing the interaction of each of the beverage consumers to the plurality of communication means;
- assessing the utilization of information transferred by each of the plurality of messages by each of the beverage consumer;
- defining a plurality beverage consumer clusters based on the hydration development of each of the beverage consumer, based on the physical conditions and/or location of each of the beverage consumer, based on the interaction of each of the beverage consumer to the plurality of communication channels and based on the utilization of information by each of the beverage consumers.

15. A method for defining types of beverage consumers implemented by a computer, comprising the following steps:

- assessing the hydration development of a plurality of beverage consumers by monitoring the volume of beverage consumed by each of the beverage consumers in at least one time interval and the effective hydration loss of each of the beverage consumers within a plurality of time intervals of a predetermined time range comprising a plurality of days;
- assessing the physical conditions and/or location of each of the beverage consumers by assessing at least the physical activity of each of the beverage consumers and the weather in the environment of each of the beverage consumers;
- sending a plurality of messages of a plurality of types to each of the beverage consumers by at least one communication means;
- assessing the interaction of each of the beverage consumers to the plurality of communication means;
- assessing the utilization of information transferred by each of the plurality of messages by each of the beverage consumer; and
- defining a plurality beverage consumer clusters based on the hydration development of each of the beverage consumer, based on the physical conditions and/or location of each of the beverage consumer, based on the interaction of each of the beverage consumer to the plurality of communication channels and based on the utilization of information by each of the beverage consumers;
- wherein the step of assessing the hydration development of the plurality of beverage consumers is performed by the method according to anyone of claims 1 to 13.

16. The method according to claim 14 or 15, further comprising the following steps:

- assessing for a plurality of beverage consumers the influence of a physical condition and/or location on the hydration development; and
- storing the influence of a physical condition and/or location on the hydration development for a plurality of beverage consumers as a first classification.

17. The method according to any one of claims 14 to 16, further comprising the following steps:

- assessing for a plurality of beverage consumers the dependency of the utilization of information on the type of message and/or communication means; and
- storing the dependency of the utilization of information on the type of message and/or communication means as a second classification.

18. The method according to any one of claims 14 to 17, further comprising the following steps:

- assessing the hydration development of a single beverage consumers by monitoring the volume of beverage consumed by each of the beverage consumers in at least one time interval and the effective hydration loss of each of the beverage consumers within a plurality of time intervals of a predetermined time range comprising a plurality of days;
- assessing the physical conditions and/or location of a single beverage consumer by assessing at least the physical activity of each of the beverage consumers and the weather in the environment of each of the beverage consumers;

**EP 3 745 410 A1**

- determining the influence of a physical condition and/or location on the hydration development by reading from the first classification;
- outputting a beverage consumption suggestion depending on the hydration development and the influence of a physical condition and/or location on the hydration development read from the first classification.

19. The method according to any claim 18, further comprising the following steps:

- determining the dependency of the utilization of information on the type of message and/or communication means by reading from the second classification; and
- outputting the beverage consumption suggestion by the type of message having the best utilization of information.

**14**

Fig. 1

Fig 3

*108*

*104*

*104 a*

*110*

*112*

*114*

*Fig. 4*

V-balance

*116*

*118*

Burst threshold

Off-balance

deficiency threshold

EP 3 745 410 A1

20

200

Start — 201

Behaviour data — 202

Context data — 204

Interaction data — 206

Feedback data — 208

Join Behaviour;Context;Interaction; Feedback — 210

Cluster **unique sets** of Behaviour;Context;Interaction; Feedback — 212

List of unique sets of Behaviour;Context;Interaction; Feedback — 214

End — 216

Fig. 6

Fig. 7

300

Start ~ 301

Context data ~ 302

i = 1 ~ 304

FALSE — End ~ 308

i < = # of contexts ~ 306

TRUE

Drinking behaviour data[i] ~ 310

Is drinking behaviour context[i] dependant? ~ 312

FALSE — List of **non-relevant** contexts with context[i] ~ 320

TRUE

Rank dependancy ~ 314

List of **relevant** contexts with context[i] + ranking ~ 316

i = i + 1 ~ 318

EP 3 745 410 A1

Fig. 8

400

Start ~ 401

Interaction data ~ 402

i = 1 ~ 404

406 — i <= # of interactions

FALSE → End (408)

TRUE

Feedback data [i] (410)

Does Feedback improve with interaction[i]? (412)

FALSE → Does Feedback decline with interaction[i]? (418)

TRUE → Rank improvement (414) → List of succsessful interactions with interactions[i] + ranking (416)

TRUE → Rank decline (420) → List of unsuccsessful interactions with interactions[i] + ranking (422)

FALSE → List of neutral interactions with interactions[i] (424)

i = i + 1 (426)

EP 3 745 410 A1

22

Start ~ 502

Behaviour data ~ 504

Context data ~ 506

Is context in **relevant** contexts list? ~ 508

FALSE

TRUE

Retain original drinking suggestion    514

Modify drinking suggestion ~ 510

Drinking suggestion ~ 512

End ~ 516

500

Fig. 9

Europäisches
Patentamt

European
Patent Office

Office européen
des brevets

# EUROPEAN SEARCH REPORT

Application Number

EP 19 17 6699

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2018/099839 A1 (NESTEC SA [CH])<br>7 June 2018 (2018-06-07)<br>* abstract *<br>* claims 21-34, 36-40 *<br>* paragraph [0003] - paragraph [0019] *<br>* paragraph [0056] - paragraph [0063] *<br>----- | 1-13<br><br>1 | INV.<br>G16H20/60<br>G16H50/20 |
| Y | WO 2016/122804 A1 (EMPIRE TECHNOLOGY DEV LLC [US]) 4 August 2016 (2016-08-04)<br>* the whole document *<br>----- | 1 | |
| X | WO 2019/046596 A1 (PERFORMANCE ATHLYTICS [US]) 7 March 2019 (2019-03-07)<br>* abstract; figures 1A-1D, 2, 6 *<br>* paragraph [0016] - paragraph [0041] *<br>* paragraph [0048] - paragraph [0054] *<br>----- | 14-19 | |

TECHNICAL FIELDS
SEARCHED (IPC)

G16H

The present search report has been drawn up for all claims

| Place of search | Date of completion of the search | Examiner |
|---|---|---|
| Munich | 12 May 2020 | Kutzarova, Iskrena |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.......................................................

& : member of the same patent family, corresponding
document

EPO FORM 1503 03.82 (P04C01)

Europäisches
Patentamt

European
Patent Office

Office européen
des brevets

**Application Number**

EP 19 17 6699

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

**Europäisches Patentamt**
**European Patent Office**
**Office européen des brevets**

## LACK OF UNITY OF INVENTION
## SHEET B

Application Number

EP 19 17 6699

The Search Division considers that the present European patent application does not comply with the requirements of unity of invention and relates to several inventions or groups of inventions, namely:

1. claims: 1-13

    Group 1: claims: 1-13The first group of inventions is directed at a method of monitoring a beverage consumption of a human.
    ---

2. claims: 14-19

    Group 2: claims 14-19The second group of inventions is directed at a method defining types of beverage consumers.
    ---

**ANNEX TO THE EUROPEAN SEARCH REPORT
ON EUROPEAN PATENT APPLICATION NO.**

EP 19 17 6699

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2020

| Patent document cited in search report | | Publication date | Patent family member(s) | | Publication date |
|---|---|---|---|---|---|
| WO 2018099839 | A1 | 07-06-2018 | EP | 3549136 A1 | 09-10-2019 |
| | | | US | 2019279758 A1 | 12-09-2019 |
| | | | WO | 2018099839 A1 | 07-06-2018 |
| WO 2016122804 | A1 | 04-08-2016 | US | 2016220184 A1 | 04-08-2016 |
| | | | WO | 2016122804 A1 | 04-08-2016 |
| WO 2019046596 | A1 | 07-03-2019 | CA | 3074278 A1 | 07-03-2019 |
| | | | WO | 2019046596 A1 | 07-03-2019 |

EPO FORM P0459

For more details about this annex : see Official Journal of the European Patent Office, No. 12/82

**EP 3 745 410 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016090235 A1
- DE 202010006679 U1
- WO 9406547 A1
- US 20130304265 A1